(19) Europäisches Patentamt
European Patent Office
Office européen des brevets

(11) Veröffentlichungsnummer: **0 074 014**
**B1**

(12) # EUROPÄISCHE PATENTSCHRIFT

(45) Veröffentlichungstag der Patentschrift: **27.06.84**

(51) Int. Cl.³: **C 07 C 93/06**, A 61 K 31/13

(21) Anmeldenummer: **82107750.0**

(22) Anmeldetag: **24.08.82**

(54) **2-(2'-Hydroxy-3'-(1,1-dimethylpropylamino)-propoxy)-beta-phenylpropiophenon, seine Säureadditionssalze, Verfahren zu seiner Herstellung und Arzneimittel.**

(30) Priorität: **25.08.81 DE 3133814**

(43) Veröffentlichungstag der Anmeldung: **16.03.83 Patentblatt 83/11**

(45) Bekanntmachung des Hinweises auf die Patenterteilung: **27.06.84 Patentblatt 84/26**

(84) Benannte Vertragsstaaten: **AT BE CH DE FR GB IT LI LU NL SE**

(56) Entgegenhaltungen: **DE - A - 2 001 431**

(73) Patentinhaber: **Helopharm W. Petrik & Co.KG., Waldstrasse 23-25, D-1000 Berlin 51 (DE)**

(72) Erfinder: **Petrik, Gerd, Cosmarweg 129, D-1000 Berlin 20 (DE)**
Erfinder: **Sachse, Rolf, Pionierstrasse 163b, D-1000 Berlin 20 (DE)**

(74) Vertreter: **Vossius Vossius Tauchner Heunemann Rauh, Siebertstrasse 4 P.O. Box 86 07 67, D-8000 München 86 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



BUNDESDRUCKEREI BERLIN

**Beschreibung**

Die Erfindung betrifft das neue 2-[2'-Hydroxy-3'-(1,1-dimethylpropylamino)-propoxy]-β-phenylpropiophenon der Formel I

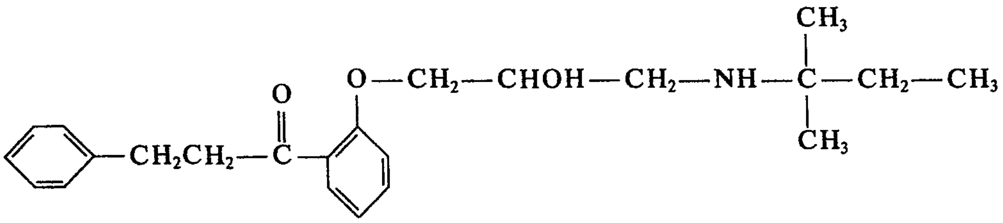

(I)

und seine Säureadditionssalze sowie ein Verfahren zu seiner Herstellung. Die Erfindung betrifft ferner Arzneimittel, die die Verbindung der Formel I oder deren Säureadditionssalz, vorzugsweise ein physiologisch verträgliches Säureadditionssalz, enthalten. Die Verbindung wird nachstehend auch mit Diprafenon bezeichnet.

Aus der DE-PS 2 001 431 sind 2-(2'-Hydroxy-3'-alkylaminopropoxy)-β-phenylpropiophenone der allgemeinen Formel

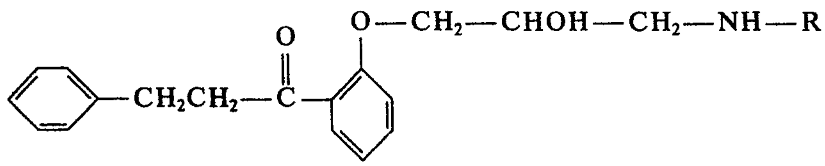

sowie deren Säureadditionssalze bekannt. Der Rest R bedeutet in dieser Formel die n-Propylgruppe

$(-CH_2-CH_2-CH_3)$,

die n-Butylgruppe

$(-CH_2-CH_2-CH_2-CH_3)$,

die 1-Methylpropylgruppe

$(-CH(CH_3)-CH_2-CH_3)$

oder die tert.-Butylgruppe

$(-C(CH_3)_3)$.

In dieser Patentschrift ist auch ein Verfahren zur Herstellung dieser Verbindungen beschrieben. Diese bekannten Verbindungen und ihre Salze sind Arzneistoffe. Untersuchungen am isolierten Meerschweinchenherzen (Methode nach Langendorff) haben gezeigt, daß diese Verbindungen eine signifikante Erhöhung des Koronarflusses bewirken. Ferner zeigt als einzige der beschriebenen Verbindungen die n-Propylaminoverbindung ($R = n-C_3H_7$; nachstehend mit Propafenon bezeichnet) zusätzlich eine antiarrhythmische Wirksamkeit. Die antiarrhythmische Wirkung des Propafenons in Tierversuchen (Hunde, Katzen und Kaninchen) mit Modellarrhythmien zeigt sich nach intravenöser (1 mg/kg) und oraler (2—10 mgKg) Applikation; vgl. H.-J. Hapke und E. Prigge, Arzneim.-Forsch. (Drug Res.) 26, 10, 1849—1857 (1976).

Die Modellarrhythmien wurden durch folgende Maßnahmen ausgelöst: Infusion von Adrenalin plus Chloroform-Inhalation oder Infusion von Digoxin, Calciumchlorid oder Aconitin, und durch Okklusion der linken Koronararterie. In mittleren (1—3 mg/kg) und höheren (5 mg/kg) intravenösen Dosen wirkt Propafenon blutdrucksenkend, negativ inotrop und koronarerweiternd. Die Herzfrequenz wird in antiarrhythmisch wirkenden Dosen nicht wesentlich geändert.

Propafenon wird inzwischen in der Humanmedizin als Antiarrhythmikum mit lokalanästhetischer, chinidinartiger, aber auch β-Rezeptoren-blockierender Wirkung zur Therapie von Herzrhythmusstörungen eingesetzt; vgl. zum Beispiel W. Baedeker, G. Klein und G. Ertl, Herz-Kreislauf, Zeitschrift für Kardiologie und Angiologie, Bd. 11, 330 (1979).

Untersuchungen an den drei anderen, aus der deutschen Patentschrift 2 001 431 bekannten 2-(2'-Hydroxy-3'-alkylaminopropoxy)-β-phenylpropiophenon-Verbindungen, nämlich der n-Butylamino-, der 1-Methylpropylamino- und der tert.-Butylamino-Verbindung, haben ergeben, daß diese Verbindungen bei mittleren (1—3 mg/kg) intravenösen Dosen praktisch keine antiarrhythmische Wirkung in Tierversuchen mit Modellarrhythmien zeigen.

Der Erfindung liegt die Aufgabe zugrunde, ein neues 2-(2'-Hydroxy-3'-alkylaminopropoxy)-β-phenylpropiophenon und dessen Säureadditionssalze zur Verfügung zu stellen, das sich durch eine wesentlich stärkere antiarrhythmische, aber keine wesentlich höhere toxische Wirkung auszeichnet als das Propafenon. Eine weitere Aufgabe der Erfindung ist es, ein Verfahren zur Herstellung dieser Verbindung und ihrer Säureadditionssalze zur Verfügung zu stellen. Schließlich ist es eine Aufgabe der Erfindung, Arzneimittel zur Therapie von Herzrhythmusstörungen zur Verfügung zu stellen, welche diese Verbindung oder ihr Säureadditionssalz, vorzugsweise ein physiologisch verträgliches Säureadditionssalz, enthalten.

Die Erfindung betrifft somit das 2-[2'-Hydroxy-3'-(1,1-dimethylpropylamino)-propoxy]-β-phenylpropiophenon (Diprafenon) der vorstehend angegebenen Formel I und seine Säureadditionssalze.

Die Erfindung betrifft ferner ein Verfahren zur Herstellung der Verbindung der Formel I und ihrer Säureadditiionssalze.

Die Verbindung der Formel I kann nach den folgenden Verfahren hergestellt werden:

Durch Umsetzung eines Phenoläthers der allgemeinen Formel II

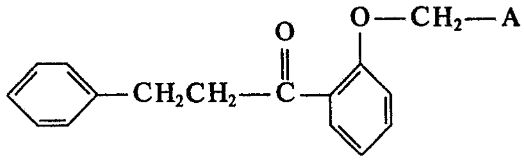

(II)

in der A den Rest

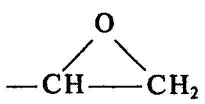

oder $-CHOH-CH_2-B$ bedeutet und B eine nucleofuge Abgangsgruppe ist, mit dem 1,1-Dimethylpropylamin der Formel III

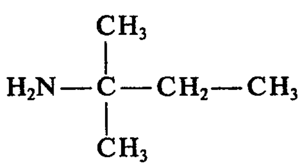

(III)

Gegebenenfalls wird die erhaltene 1,1-Dimethylpropylamino-Verbindung mit einer Säure in ein Säureadditionssalz überführt. Die Umsetzung kann beispielsweise nach den in der DE-PS 2 001 431 und DE-OS 2 007 751 beschriebenen Verfahren erfolgen.

Die Abgangsgruppe B stellt bevorzugt ein Halogenatom, insbesondere ein Chlor-, Brom- oder Jodatom, dar. Weiterhin kommen aromatische oder aliphatische Sulfonsäurereste in Betracht, wie der p-Toluolsulfonsäure-, der p-Brombenzolsulfonsäure- oder der Methansulfonsäurerest.

Die Umsetzung wird bei Temperaturen von 10 bis 120° C, d. h. bei Raumtemperatur oder bei höheren Temperaturen, zweckmäßig bei Temperaturen von 50 bis 120° C, unter Atmosphärendruck oder in einem geschlossenen Gefäß unter erhöhtem Druck durchgeführt.

Die Ausgangsverbindungen der Formel II und III können ohne Verdünnungs- oder Lösungsmittel umgesetzt werden. Zweckmäßigerweise werden die Umsetzungen jedoch durchgeführt in Gegenwart eines inerten Verdünnungs- oder Lösungsmittels, beispielsweise eines niederen Alkohols mit 1 bis 4 C-Atomen, wie Methanol, Äthanol oder Propanol, vorzugsweise Isopropanol oder Äthanol, eines niederen gesättigten Dialkyläthers, Dialkylglykoläthers oder cyclischen Äthers, wie Diäthyläther, 1,2-Dimethoxyäthan, Tetrahydrofuran oder Dioxan, eines Benzolkohlenwasserstoffs, wie Benzol selbst oder eines Alkylbenzols, insbesondere Toluol oder Xylol, oder eines aliphatischen Kohlenwasserstoffs, wie Hexan, Heptan oder Octan, Dimethylsulfoxid oder in Gegenwart von Wasser oder Mischungen der genannten Lösungsmittel.

Auch ist das in überschüssiger Menge verwendete 1,1-Dimethylpropylamin (2-Methyl-2-aminobutan) gegebenenfalls als Verdünnungs- oder Lösungsmittel geeignet.

Bevorzugte Lösungsmittel bei Umsetzungen von Verbindungen der Formel II, worin A den Rest

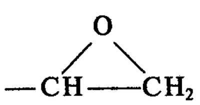

bedeutet, mit dem Amin der Formel III sind niedere Alkohole, insbesondere Äthanol oder Isopropanol, wobei die Umsetzung bevorzugt bei Temperaturen von 50 bis 120° C durchgeführt wird. Gegebenenfalls kann die Umsetzung in einem geschlossenen Gefäß unter Druck durchgeführt werden.

Bei der nucleophilen Substitution des Restes B sind als Lösungsmittel ein cyclischer aliphatischer Äther, insbesondere Tetrahydrofuran oder Dioxan, oder Dimethylsulfoxid, und Temperaturen von 90 bis 120°C bevorzugt. Gegebenenfalls wird die Reaktion in Gegenwart einer katalytischen Menge Natrium- oder Kaliumjodid durchgeführt.

Der Phenoläther der allgemeinen Formel II kann gegebenenfalls auch in Form einer Mischung eines Epoxids mit einem Halogenhydrin verwendet werden, da bei der technischen Herstellung der Ausgangsverbindungen unter Umständen derartige Gemische entstehen können.

Bei einer zweckmäßigen Ausführungsform zur nucleophilen Substitution des Restes B durch das Amin der Formel III wird die Umsetzung in Gegenwart einer Base als säurebindendes Mittel durchgeführt. Bevorzugte Basen sind Alkalimetallhydroxide, -carbonate, -hydrogencarbonate, -alkoholate, insbesondere Methylate und Äthylate, oder ein tertiäres Amin, wie Pyridin, oder ein Trialkylamin, wie Trimethylamin oder Triäthylamin. Von den Alkaliverbindungen kommen insbesondere die des Natriums und Kaliums in Betracht. Dabei wird die Base in stöchiometrischer Menge oder in geringem Überschuß verwendet.

Gegebenenfalls kann das zur Umsetzung verwendete Amin im Überschuß gleichzeitig als säurebindendes Mittel verwendet werden.

Die vollständige Umsetzung hängt von der Reaktionstemperatur ab und ist im allgemeinen innerhalb von 2 bis 15 Stunden beendet. Das Reaktionsprodukt kann in an sich üblicher Weise gewonnen werden, z. B. durch Filtration oder Abdestillieren des Verdünnungsmittels aus dem Reaktionsgemisch. Eine Reinigung der erhaltenen Verbindung erfolgt in üblicher Weise, beispielsweise durch Umkristallisation aus einem Lösungsmittel, Überführen in ein Säureadditionssalz oder durch Säulenchromatographie.

Der Phenoläther der allgemeinen Formel II kann durch Alkylierung von 2-Hydroxy-$\beta$-phenylpropiophenon der Formel IV

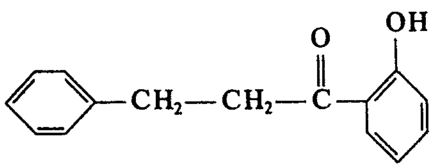

(IV)

mit einem Epihalogenhydrin oder einem $\alpha,\omega$-Dihalogen-2-propanol erhalten werden. Als Epihalogenhydrine kommen Epichlorhydrin, Epibromhydrin und Epijodhydrin und als $\alpha,\omega$-Dihalogen-2-propanol kommen insbesondere 1,3-Dichlor-2-propanol und 1,3-Dibrom-2-propanol in Betracht.

Die Umsetzung der Verbindungen IV zur Herstellung der Ausgangsverbindungen der Formel II wird zweckmäßig bei Temperaturen von 0 bis 120°C und unter Normaldruck oder in einem geschlossenen Gefäß unter erhöhtem Druck durchgeführt. Als Lösungs- oder Verdünnungsmittel werden zweckmäßig ein niederes aliphatisches Keton, wie Aceton, Methyläthylketon oder Methylisobutylketon, ein niederer Alkohol mit 1 bis 4 C-Atomen, wie Methanol, Äthanol, Propanol oder Butanol, ein niederer aliphatischer oder cyclischer Äther, wie Diäthyläther, Tetrahydrofuran oder Dioxan, ein Dialkylformamid, wie Dimethylformamid oder Diäthylformamid, oder Dimethylsulfoxid oder Hexamethylphosphorsäuretriamid oder überschüssiges Alkylierungsmittel verwendet.

Bevorzugt werden die Umsetzungen in Gegenwart einer Base als säurebindendes Mittel durchgeführt. Geeignete Basen sind Alkalimetallcarbonate, -hydrogencarbonate, -hydroxide, -hydride oder -alkoholate, insbesondere des Natriums und Kaliums, basische Oxide, wie Aluminiumoxid oder Calciumoxid, organische tertiäre Basen, wie Pyridin, niedere Trialkylamine, wie Trimethyl- oder Triäthylamin, oder Piperidin. Dabei können die Basen im Verhältnis zum eingesetzten Alkylierungsmittel in katalytischer Menge oder in stöchiometrischer Menge bzw. in geringem Überschuß verwendet werden.

Bevorzugt wird 2-Hydroxy-$\beta$-phenylpropiophenon mit Epichlorhydrin, Epibromhydrin oder 1,3-Dibrompropanol-2 in einem polaren, aprotischen Lösungsmittel, insbesondere Dimethylsulfoxid, in Gegenwart von mindestens einem Moläquivalent Base, insbesondere Natriumhydrid, bezogen auf das Alkylierungsmittel, bei Temperaturen von 0 bis 50°C umgesetzt.

Die Ausgangsverbindung der Formel IV, d. h. das 2-Hydroxy-$\beta$-phenylpropiophenon, und seine Herstellung ist bekannt.

Die Verbindung der Formel I weist am Kohlenstoffatom in der 2-Stellung der aliphatischen Seitenkette ein Chiralitätszentrum auf. Im allgemeinen wird sie als Racemat erhalten, das durch bekannte Methoden, beispielsweise durch Bildung diastereomerer Salze mit optisch aktiven Hilfssäuren, wie Dibenzoylweinsäure, Campher-10-sulfonsäure, Ditoluylweinsäure oder 3-Brom-campher-8-sulfonsäure, in die optisch aktive Antipoden gespalten werden kann.

Gegebenenfalls wird die erhaltene erfindungsgemäße Verbindung der Formel I in ein Säureadditionssalz, vorzugsweise in ein Salz einer physiologisch verträglichen Säure überführt. Übliche physiologisch verträgliche anorganische und organische Säuren sind beispielsweise Salzsäure, Bromwasserstoffsäure, Phosphorsäure, Schwefelsäure, Oxalsäure, Maleinsäure, Fumarsäure, Milchsäure, Weinsäure, Äpfelsäure, Citronensäure, Salicylsäure, Adipinsäure und Benzoesäure. Weitere verwendbare Säuren sind beschrieben z. B. in Fortschritte der Arzneimittelforschung, Bd. 10, Seiten 224—225, Birkhäuser Verlag, Basel und Stuttgart, 1966, und Journal of Pharmaceutical Sciences, Bd. 66, Seiten 1—5 (1977). Bevorzugt ist Salzsäure.

Die Säureadditionssalze werden in der Regel in an sich bekannter Weise durch Mischen der freien Base oder deren Lösungen mit der entsprechenden Säure oder deren Lösungen in einem organischen Lösungsmittel, beispielsweise einem niederen Alkohol, wie Methanol, Äthanol, n-Propanol oder Isopropanol, oder einem niederen Keton, wie Aceton, Methyläthylketon oder Methyl-isobutylketon, oder einem Äther, wie Diäthyläther, Tetrahydrofuran oder Dioxan, erhalten. Zur besseren Kristallabscheidung können auch Mischungen der genannten Lösungsmittel verwendet werden. Darüber hinaus können pharmazeutisch vertretbare wäßrige Lösungen von Säureadditionsverbindungen der Verbindung der Formel I in einer wäßrigen Säurelösung hergestellt werden.

Die Säureadditionssalze der Verbindung der Formel I können in an sich bekannter Weise, z. B. mit Alkalien oder Ionenaustauschern, in die freie Base übergeführt werden. Von der freien Base lassen sich durch Umsetzung mit anorganischen oder organischen Säuren, insbesondere solchen, die zur Bildung von therapeutisch verwendbaren Salzen geeignet sind, weitere Salze gewinnen. Diese oder auch andere Salze der neuen Verbindung, wie z. B. das Pikrat, können auch zur Reinigung der freien Base dienen, indem man die freie Base in ein Salz überführt, dieses abtrennt und aus dem Salz wiederum die Base freisetzt.

Gegenstand der vorliegenden Erfindung sind auch Arzneimittel zur oralen, rektalen, intravenösen oder intramuskulären Applikation, die neben üblichen Träger- und Verdünnungsmitteln die Verbindung der Formel I oder deren Säureadditionssalz als Wirkstoff enthalten, sowie die Verwendung der neuen Verbindung und ihrer physiologisch verträglichen Salze bei der Behandlung von Herzrhythmusstörungen.

Die Arzneimittel der Erfindung werden mit den üblichen festen oder flüssigen Trägerstoffen oder Verdünnungsmitteln und den üblicherweise verwendeten pharmazeutisch-technischen Hilfsstoffen entsprechend der gewünschten Applikationsart mit einer geeigneten Dosierung in bekannter Weise hergestellt. Die bevorzugten Zubereitungen bestehen in einer Darreichungsform, die zur oralen Applikation geeignet ist. Solche Darreichungsformen sind beispielsweise Tabletten, Filmtabletten, Dragees, Kapseln, Pillen, Pulver, Lösungen oder Suspensionen oder Depotformen.

Selbstverständlich kommen auch parenterale Zubereitungen, wie Injektionslösungen, in Betracht. Weiterhin seien als Zubereitungen beispielsweise auch Suppositorien genannt.

Entsprechende Tabletten können beispielsweise durch Mischen des Wirkstoffs mit bekannten Hilfsstoffen, beispielsweise inerten Verdünnungsmitteln wie Dextrose, Zucker, Sorbit, Mannit, Polyvinylpyrrolidon, Sprengmittel wie Maisstärke oder Alginsäure, Bindemitteln wie Stärke oder Gelatine, Gleitmitteln wie Magnesiumstearat oder Talk und/oder Mitteln zur Erzielung eines Depoteffektes wie Carboxypolymethylen, Carboxymethylcellulose, Celluloseacetatphthalat oder Polyvinylacetat, erhalten werden. Die Tabletten können auch aus mehreren Schichten bestehen.

Entsprechend können Dragees durch Überziehen von analog den Tabletten hergestellten Kernen mit üblicherweise in Drageeüberzügen verwendeten Mitteln, beispielsweise Polyvinylpyrrolidon oder Schellack, Gummiarabicum, Talk, Titandioxid oder Zucker, hergestellt werden. Dabei kann auch die Drageehülle aus mehreren Schichten bestehen, wobei die oben bei den Tabletten erwähnten Hilfsstoffe verwendet werden können.

Lösungen oder Suspensionen mit dem erfindungsgemäßen Wirkstoff können zusätzlich geschmacksverbessernde Mittel wie Saccharin, Cyclamat oder Zucker sowie z. B. Aromastoffe wie Vanillin oder Orangenextrakt, enthalten. Sie können außerdem Suspendierhilfsstoffe wie Natriumcarboxymethylcellulose oder Konservierungsstoffe wie p-Hydroxybenzoate, enthalten. Wirkstoffe enthaltende Kapseln können beispielsweise hergestellt werden, indem man den Wirkstoff mit einem inerten Träger, wie Milchzucker oder Sorbit, mischt und in Gelatinekapseln einkapselt.

Geeignete Suppositorien lassen sich beispielsweise durch Vermischen mit dafür vorgesehenen Trägermitteln wie Neutralfetten oder Polyäthylenglykol bzw. deren Derivaten, herstellen.

Die Einzeldosierung liegt beim Menschen

| | |
|---|---|
| für die orale Anwendung zwischen | 0,5—5 mg/kg |
| für die i. v. Anwendung zwischen | 0,05—2 mg/kg |
| für die i. m. Anwendung zwischen | 0,1—3 mg/kg |
| für die rektale Anwendung zwischen | 0,5—10 kmg/kg. |

Überraschenderweise hat die Verbindung der Formel I, d. h. das 2-[2'-Hydroxy-3'-(1,1-dimethylpropylamino)-propoxy]-$\beta$-phenylpropiophenon (Diprafenon) unter den vorstehend von H.-J. Hapke und E. Prigge, Arzneim.-Forsch. (Drug Res.), a. a. O. beschriebenen Bedingungen bei Modellarrhythmien eine antiarrhythmische Wirksamkeit bereits bei 0,05 mg/kg bei intravenöser Applikation, d. h. also bei einem Zwanzigstel der erforderlichen Dosis der Vergleichssubstanz Propafenon.

Diese überraschend hohe antiarrhythmische Wirksamkeit ist nicht begleitet von einer ebenso großen Toxizitätssteigerung. Dies ergibt sich aus einem Vergleich der $LD_{50}$-Werte bei Ratte und Hund, die in Tabelle I zusammengefaßt sind.

Tabelle I

| | $LD_{50}$ (mg/kg) Propafenon-HCl | Diprafenon-HCl |
|---|---|---|
| Ratte i. v. | 18,8 | 16,9 |
| Ratte oral | 760 | 1080 |
| Hund i. v. | 15,0 | 10,0 |

Hieraus resultiert dementsprechend eine Erweiterung des therapeutischen Quotienten um das Zehn- bis Zwanzigfache bei Diprafenon gegenüber Propafenon.

Die antiarrhythmische Wirksamkeit der Verbindung der Formel I wurde an Hunden nach H.-J. Hapke und E. Prigge, Arzneim.-Forsch. (Drug Res.), a.a.O. geprüft. Es wurde mit Hilfe einer Chloroform-Infusion und Adrenalin-Infusion eine Modellarrhythmie erzeugt, die im wesentlichen durch ventrikuläre Extrasystolen bei absoluter Arrhythmie gekennzeichnet war. Unmittelbar nach Eintreten dieser Rhythmusstörungen wurde die erfindungsgemäße Verbindung als wäßrige Lösung des Hydrochlorids intravenös injiziert.

Bereits eine intravenöse Dosis von 0,05 mg/kg war bei allen damit behandelten Hunden innerhalb von 45 Sekunden voll wirksam, d. h., es stellte sich im Elektrokardiogramm (EKG) ein normaler Sinusrhythmus ein.

Ebenfalls an Hunden konnte auch nach oraler Gabe die ausgezeichnete antiarrhythmische Wirksamkeit festgestellt werden. Durch Okklusion eines Astes der Herzkranzgefäße wurden Rhythmusstörungen erzeugt. Eine intragastrale Applikation der erfindungsgemäßen Verbindung in Form des Hydrochlorids erfolgte am nächsten Tag. 10 Minuten nach der Applikation von 10 mg/kg war unverändert eine Extrasystole nachweisbar. 30 Minuten nach der Gabe wurden keine Extrasystolen mehr festgestellt. Es wurde ein normales EKG bis zur Dauer von 24 Stunden beobachtet.

In Tabelle II sind weitere Ergebnisse pharmakologischer Untersuchungen an Hunden mit der Verbindung der Erfindung als Hydrochlorid und mit Propafenon-HCl zusammengestellt. Zur Versuchsmethodik vgl. H.-J. Hapke und E. Prigge, Arzneim.-Forsch. (Drug Res.), a.a.O.

Alle diese aufgeführten Versuchsergebnisse lassen erwarten, daß sich auch in der Humanmedizin die Verbindung der Formel I (Diprafenon) in derselben Weise wie Propafenon als Antiarrhythmikum einsetzen läßt, jedoch mit wesentlich geringerer Dosierung und damit geringerer Toxizität.

Tabelle II

| Kreislaufparameter Sonstige Prüfkriterien | Testverbindung | Dosierung (mg/kg) i. v. 0,05 | 0,1 | 0,2 | 0,3 | 0,5 | 1,0 | 3,0 | 5,0 | 10,0 |
|---|---|---|---|---|---|---|---|---|---|---|
| Systolischer Blutdruck (A. femoralis), mm Hg | D | | 0 | 0 | | −13 | −17 | −45 | | |
| | P | | | | 0 | | 0 | −30 | | −65 |
| Diastolischer Blutdruck (A. femoralis), mm Hg | D | | 0 | 0 | | −16 | −24 | −39 | | |
| | P | | | | 0 | | 0 | −40 | | −60 |
| Herzschlagfrequenz Veränderung $min^{-1}$ | D | | 0 | 0 | | −8 | −22 | −24 | | |
| | P | | | | 0 | | 0 | 0 | | |
| Linksventrikulärer Spitzendruck, mm Hg | D | | 0 | 0 | | 0 | 0 | −46 | | |
| | P | | | | 0 | | −5 | −25 | −42 | −70 |
| Maximale Druckanstiegsgeschwindigkeit (dp/dt) % | D | | 0 | 0 | | 0 | 0 | −30 | | |
| | P | | | | 0 | | 0 | −29 | −47 | −62 |
| Peripheres Blutflußvolumen, % (A. femoralis) | D | | 0 | 0 | | 0 | 0 | 0 | | |
| | P | | | | 0 | | +10 | +36 | | |
| Koronarflußvolumen, % | D | 0 | | | 0 | | 0 | +56 | | |
| | P | | | | | | +14 | +30 | +43 | +67 |
| Antiarrhythmischer Effekt ($CHCl_3$-Adrenalin-Modellarrhythmie) | D | + | + | + | | + | + | | | |
| | P | 0 | 0 | 0 | 0 | 0 | + | + | | |

D = Diprafenon,
P = Propafenon,
0 = nicht signifikant unterschiedlich zum Ausgangswert,
+ = signifikante Wirkung.

Es ist überraschend und war nicht zu erwarten, daß die erfindungsgemäße Verbindung trotz ihrer großen Ähnlichkeit mit den bekannten 2-(2'-Hydroxy-3'-alkylaminopropoxy)-$\beta$-phenylpropiophenon-Verbindungen in vergleichbaren Dosen keine Wirkung auf die periphere und koronare Durchblutung hat und trotz etwa gleichartiger hämodynamischer Wirkungen eine etwa zwanzigfach stärkere Wirkung bei Herzrhythmusstörungen aufweist als Propafenon. Die Verbindung der Formel I kann deshalb in erheblich geringerer Dosierung als Propafenon eingesetzt werden. Dies hat in der Therapie der Herzrhythmusstörungen eine wesentliche Verminderung von Nebenwirkungen zur Folge.

Bemerkenswert ist ferner, daß im Gegensatz zu Propafenon die Verbindung der Erfindung schon bei einer Dosis von 0,5 mg/kg i. v. eine Verminderung der Herzschlagfrequenz bewirkt. Dies ist bei tachykarden Herzrhythmusstörungen erwünscht.

Die Beispiele erläutern die Erfindung.

## Beispiel 1

### a) Herstellung von 2-(2',3'-Epoxypropoxy)-$\beta$-phenylpropiophenon

22,6 g (0,1 Mol) 2-Hydroxy-$\beta$-phenylpropiophenon werden mit 150 ml 1-Chlor-2,3-epoxypropan in Lösung gebracht. Nach Zusatz von 12 g Kaliumcarbonat wird das Reaktionsgemisch unter Rühren und Rückflußkühlung so lange erhitzt, bis mit Hilfe der Hochdruckflüssigkeitschromatographie eine komplette Umsetzung nachgewiesen ist. Dann wird das Reaktionsgemisch abkühlen gelassen. Anschließend wird das entstandene Kaliumchlorid abfiltriert. Das Filtrat wird unter vermindertem Druck eingedampft. Dabei wird das überschüssige 1-Chlor-2,3-epoxypropan vom entstandenen 2-(2',3'-Epoxypropoxy)-$\beta$-phenylpropiophenon abdestilliert. Das Rohprodukt hinterbleibt als gelblich gefärbtes Öl, das bei längerem Stehen bei Raumtemperatur erstarrt (Ausbeute 28 g; 98% d. Th.). Eine Reinigung des Produktes für die nächste Stufe ist nicht erforderlich. Die reine Substanz, die durch Umkristallisieren aus der 4fachen Menge Methanol erhalten wird, hat einen Schmelzpunkt von 56° C.

$C_{18}H_{18}O_3$;

| | | |
|---|---|---|
| ber.: | C 76,58 | H 6,42 |
| gef.: | C 76,92 | H 6,38 |

### b) Herstellung von 2-[2'-Hydroxy-3'-(1,1-dimethylpropylamino)-propoxy]-$\beta$-phenylpropiophenon-hydrochlorid (Diprafenon-HCl)

28 g (0,1 Mol) der nach Beispiel 1a) erhaltenen Verbindung werden in 100 ml Methanol gelöst und mit 26 g 1,1-Dimethylpropylamin (2-Methyl-2-aminobutan) versetzt. Anschließend wird das Gemisch 4 Stunden unter Rühren und Rückflußkühlung erhitzt. Danach wird das Reaktionsgemisch in einem Rotationsverdampfer eingedampft. Der erhaltene Rückstand wird unter Erwärmen in 100 ml Isopropanol gelöst und mit konzentrierter 36%iger Salzsäure auf pH 1 eingestellt. Das Gemisch wird bei Raumtemperatur stehengelassen. Dabei kristallisiert das Hydrolchlorid aus. Das erhaltene Rohprodukt (ca. 36 g) wird aus der gleichen Volumenmenge Isopropanol umkristallisiert. Man erhält etwa 33,0 g (81,3% d. Th.) des Hydrochlorids als weiße kristalline Substanz vom Schmelzpunkt 130—131° C. Die Analysenwerte der reinen Substanz entsprechen der Theorie.

$C_{23}H_{32}NO_3Cl$;

| | | | |
|---|---|---|---|
| ber.: | C 68,05 | H 7,94 | N 3,45 |
| gef.: | C 67,97 | H 7,91 | N 3,72 |

## Beispiel 2

Bei der Umsetzung von 2-(2'-Hydroxy-3'-brompropoxy)-$\beta$-phenylpropiophenon mit einer äquivalenten Menge 2-Methyl-2-aminobutan in Gegenwart von Dimethylformamid als Lösungsmittel und Natriumcarbonat als Säureakzeptor durch mehrstündiges Rückflußkochen wird nach dem Aufarbeiten des Reaktionsgemisches gemäß Beispiel 1b) das Diprafenon-hydrochlorid erhalten. F. 130° C

## Beispiel 3

Beispiel 2 wird mit 2-(2'-Hydroxy-3'-chlorpropoxy)-β-phenylpropiophenon wiederholt. Es wird das Diprafenon-hydrochlorid vom F. 130—131°C erhalten.

## Beispiel 4

### Herstellungsvorschrift für Tabletten

a) Rezeptur

| | |
|---|---|
| Diprafenon-HCl | 75,00 g |
| Mikrokristalline Cellulose (Pulver, 50 µm) | 15,75 g |
| Poly-(1-vinyl-2-pyrrolidon) | 5,00 g |
| Hydroxypropylmethylcellulose 2910 | 3,75 g |
| Magnesiumstearat | 0,50 g |
| | 100,00 g |

### b) Mischen und Granulieren

Das Diprafenon-HCl wird bei Bedarf gesiebt, dann werden alle Rohstoffe außer Magnesiumstearat in einem Mischer gemischt und ebenfalls im Mischer mit einer geeigneten Menge einer Granulierflüssigkeit (z. B. Wasser oder Isopropanol-Dichlormethan 1 : 1) befeuchtet. Die feuchte Mischung wird mit einem geeigneten Sieb gesiebt, im Trockenschrank getrocknet und nochmals gesiebt. Das trockene Granulat wird mit dem Magnesiumstearat im Mischer vermengt.

c) Andeckmischung

| | |
|---|---|
| Talkum | 70,0% |
| Calciumsulfat-Hemihydrat | 26,7% |
| Hochdisperses Siliciumdioxid | 3,3% |
| | 100,0% |

d) Dragiersuspension

| | |
|---|---|
| Saccharose | 47,8% |
| Talkum | 9,6% |
| Calciumsulfat-Hemihydrat | 4,8% |
| Arabisches Gummi | 3,6% |
| Stärkesirup | 3,2% |
| Macrogol® 6000 | 2,9% |
| Titan(IV)-oxid | 1,6% |
| Natriumdodecylsulfat | 0,1% |
| dest. Wasser | 26,4% |
| | 100,0% |

### Überziehen der Drageekerne

Die Drageekerne werden zunächst im rotierenden Kessel mit der Dragierlösung angefeuchtet und dann mit so viel Andeckmischung abgepudert, bis sie wieder frei rollen. Nach dem Trocknen der Kerne wird dieser Vorgang wiederholt. Dann werden die Kerne im Trockenschrank nachgetrocknet. Danach werden die Kerne mit der Dragiersuspension schichtweise überzogen, bis das gewünschte Endgewicht erreicht ist. Jeweils nach dem Auftrag einer Schicht müssen die Kerne getrocknet werden.

### Pressen von Tabletten

Aus der nach a) hergestellten Mischung werden in einer Tablettenpresse Tabletten mit einem Gewicht zwischen 40 und 400 mg gepreßt. Die Preßkraft und der Tablettendurchmesser werden so gewählt, daß die Zerfallzeit im Testgerät nach Ph. Eur. unter 15 Minuten liegt und die Tabletten mechanisch genügend stabil sind.

Beispiel 5

Herstellungsvorschrift für Filmtabletten

A. Rezeptur

a) Tablette
(sieh Herstellungsvorschrift für Tabletten, Beispiel 4).

b) Filmüberzug Gesamtauftragsmenge 5—20% des Tablettengewichts, davon:

| | |
|---|---|
| Hydroxypropylmethylcellulose 2910 | 77% |
| Macrogol® 6000 (Weichmacher) | 23% |

B. Herstellung der Tabletten

(siehe Herstellungsvorschrift für Tabletten, Beispiel 4).

C. Herstellung der Filmtabletten

Die Bestandteile des Filmüberzugs werden in einem geeigneten Lösungsmittel (z. B. Wasser oder Äthanol/Wasser 70 : 30) gelöst. Die Tabletten werden in einer Filmcoating-Anlage mit der Lösung des Filmbildners und des Weichmachers besprüht und im Heißluftstrom getrocknet. Die Filmtabletten werden im Trockenschrank nachgetrocknet.

Beispiel 6

Herstellungsvorschrift für Dragees

A. Rezeptur

a) Drageekern
(siehe Herstellungsvorschrift für Tabletten)

b) Drageedecke
Gesamtauftragsmenge 25—100% des Kerngewichts
davon:

| | |
|---|---|
| Saccharose | 51,4% |
| Talkum | 24,0% |
| Calciumsulfat-Hemihydrat | 10,3% |
| Stärkesirup | 5,0% |
| Arabisches Gummi | 3,9% |
| Macrogol® 6000 | 2,9% |
| Titan(IV)-oxid | 1,6% |
| Hochdisperses Siliciumdioxid | 0,8% |
| Natriumdodecylsulfat | 0,1% |
| | 100,0% |

B. Herstellung der Drageekerne

(siehe Herstellungsvorschrift für Tabletten)
C. Herstellung der Dragees

Zusammensetzung der verwendeten Dragierlösung, Andeckmischung und Dragiersuspension

a) Dragierlösung

| | |
|---|---|
| Saccharose | 47,6% |
| Stärkesirup | 19,1% |
| Arabisches Gummi | 3,8% |
| dest. Wasser | 29,5% |
| | 100,0% |

## Beispiel 7

### Herstellungsvorschrift für Kapseln

#### A. Wirkstoffdosierung ab 75 mg

1. Rezeptur

| | |
|---|---|
| Diprafenon-HCl | 75,00 g |
| Mikrokristalline Cellulose-Pulver, 50 µm | 16,25 g |
| Poly-(1-vinyl-2-pyrrolidon) | 5,00 g |
| Hydroxypropylmethylcellulose 2910 | 3,75 g |
| | 100,00 g |

2. Mischen und Granulieren gemäß Beispiel 4b).

3. Abfüllen des Granulats in Kapseln
Das Granulat wird mit Hilfe einer Kapselfüllmaschine in Hartgelatinekapseln der Größe 3, 2, 1 oder 0 abgefüllt. Die Füllmenge pro Kapsel richtet sich dabei nach der gewünschten Dosierung des Wirkstoffes.

#### B. Wirkstoffdosierung 30—75 mg

1. Rezeptur

| | |
|---|---|
| Diprafenon-HCl | 30,00—75,00 g |
| Mikrokristalline Cellulose-Pulver, 50 µm | 61,25—16,25 g |
| Poly-(1-vinyl-2-pyrrolidon) | 5,00 g |
| Hydroxypropylmethylcellulose 2910 | 3,75 g |
| | 100,00 g |

Die Summe der Einwaagen von Wirkstoff und mikrokristalliner Cellulose soll immer 91,25 g betragen. Die Wirkstoffmenge beträgt das Tausendfache der Einzeldosierung.

2. Mischen und Granulieren gemäß Beispiel 4b).

3. Abfüllen des Granulats in Kapseln
Je 100 mg Granulat werden mit Hilfe einer Kapselfüllmaschine in Hartgelatinekapseln der Größe 3 oder 2 abgefüllt.

## Beispiel 8

### Herstellungsvorschrift für Ampullen

1. Rezeptur

| | |
|---|---|
| Diprafenon-HCl | 1,5 g |
| Wasser für Injektionszwecke ad | 100,0 ml |

2. Herstellung der Lösung
90% des für die gewählte Ansatzgröße benötigten Wassers werden vorgelegt; der Wirkstoff wird unter Erwärmen darin gelöst. Die Lösung wird nach dem Abkühlen auf das Endvolumen aufgefüllt.

3. Abfüllen der Lösung
Die fertige Lösung wird in Glasampullen, deren Füllmenge sich nach der gewünschten Dosierung richtet, abgefüllt. Sodann werden die Glasampullen zugeschmolzen.

4. Sterilisation
Die Ampullen werden 20 Minuten bei 120° C im Dampf sterilisiert.

Beispiel 9

Herstellungsvorschrift für Suppositorien

a. Rezeptur

| | |
|---|---|
| Diprafenon-HCl | 30—200 mg |
| Hartfett (Schmelzpunkt 35—36,5° C) ad | 2000 mg |

b. Herstellung der wirkstoffhaltigen Schmelze

Die für eine bestimmte Anzahl Suppositorien benötigte Menge Hartfett wird bei 40° C im Wasserbad geschmolzen. Der Wirkstoff wird durch ein 0,8-mm-Sieb gedrückt und in die Schmelze eingerührt, so daß eine Suspension entsteht.

c. Herstellung der Suppositorien

Die Schmelze wird auf 37—38° C abkühlen gelassen und unter ständigem Rühren in Zäpfchenformen so abgefüllt, daß das Gewicht eines Suppositoriums 2000 mg beträgt. Die Zäpfchenform wird nach dem Erstarren der Schmelze verschlossen.

**Patentansprüche für die Vertragsstaaten: BE, CH/LI, DE, FR, GB, IT, LU, NL, SE**

1. 2-[2'-Hydroxy-3'-(1,1-dimethylpropylamino)-propoxy]-*β*-phenylpropiophenon der Formel I

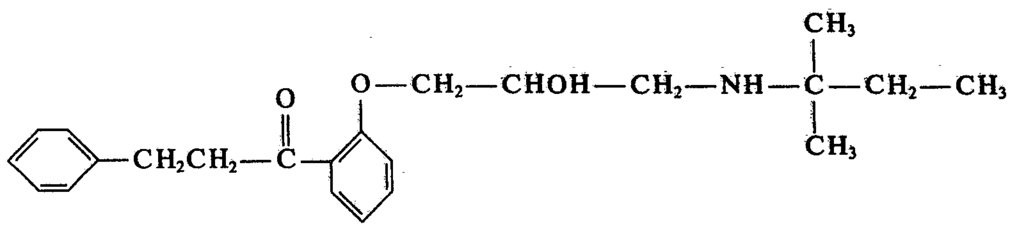

(I)

und seine Säureadditionssalze.

2. 2-[2'-Hydroxy-3'-(1,1-dimethylpropylamino)-propoxy]-*β*-phenylpropiophenon-Hydrochlorid.

3. Verfahren zur Herstellung von 2-[2'-Hydroxy-3'-(1,1-dimethylpropylamino)-propoxy]-*β*-phenylpropiophenon der Formel I

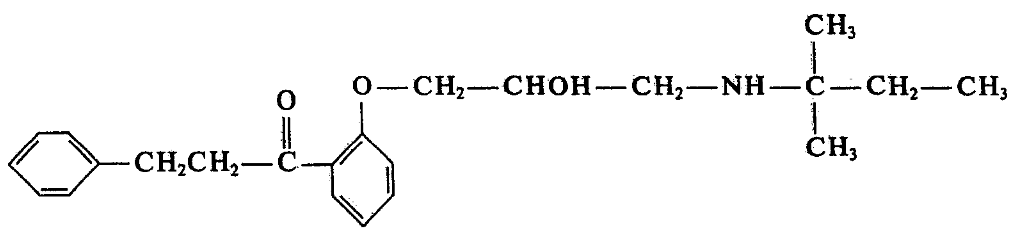

(I)

und seinen Säureadditionssalzen, dadurch gekennzeichnet, daß man einen Phenoläther der allgemeinen Formel II

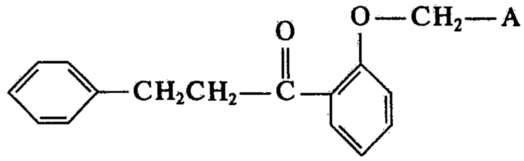

(II)

in der A den Rest

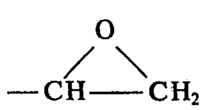

oder

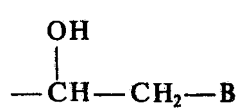

bedeutet und B eine nucleofuge Abgangsgruppe ist, mit dem 1,1-Dimethylpropylamin der Formel III

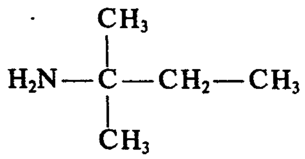

(III)

zur Umsetzung bringt und gegebenenfalls die erhaltene Verbindung der Formel I mit einer optisch aktiven Säure in die Isomeren spaltet und/oder durch Umsetzung mit einer Säure in ein Säureadditionssalz überführt.

4. Arzneimittel zur Behandlung von Herzrhythmusstörungen, gekennzeichnet durch einen Gehalt an 2-[2'-Hydroxy-3'-(1,1-dimethylpropylamino)-propoxy]-$\beta$-phenylpropiophenon oder dessen physiologisch verträglichem Säureadditionssalz.

**Patentansprüche für den Vertragsstaat AT**

1. Verfahren zur Herstellung von 2-[2'-Hydroxy-3'-(1,1-dimethylpropylamino)-propoxy]-$\beta$-phenylpropiophenon der Formel I

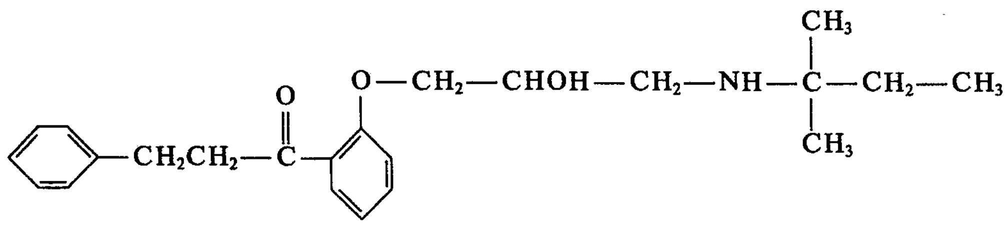

(I)

und seinen Säureadditionssalzen, dadurch gekennzeichnet, daß man einen Phenoläther der allgemeinen Formel II

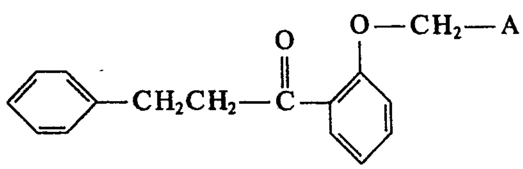

(II)

in der A den Rest

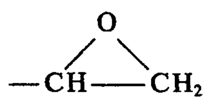

oder

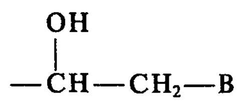

bedeutet und B eine nucleofuge Abgangsgruppe ist, mit dem 1,1-Dimethylpropylamin der Formel III

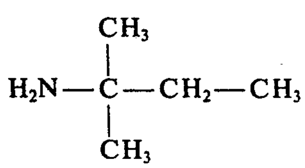

(III)

zur Umsetzung bringt und gegebenenfalls die erhaltene Verbindung der Formel I mit einer optisch aktiven Säure in die Isomeren spaltet und/oder durch Umsetzung mit einer Säure in ein Säureadditionssalz überführt.

2. Arzneimittel zur Behandlung von Herzrhythmusstörungen, gekennzeichnet durch einen Gehalt an 2-[2'-Hydroxy-3'-(1,1-dimethylpropylamino)-propoxy]-$\beta$-phenylpropiophenon oder dessen physiologisch verträglichem Säureadditionssalz.

**Claims for the contracting states BE, CH/LI, DE, FR, GB, IT, LU, NL, SE**

1. 2-[2'-hydroxy-3'-(1,1-dimethylpropylamino)-propoxy]-$\beta$-phenylpropiophenone of the formula I

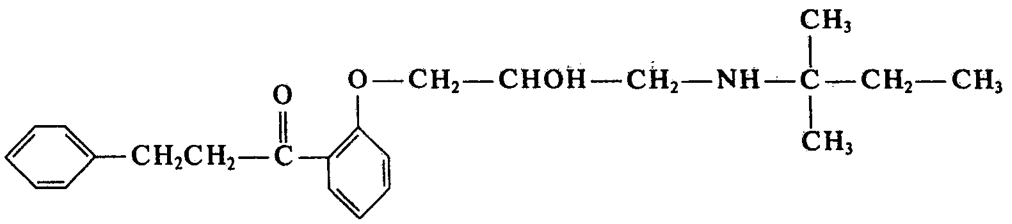

(I)

and its acid addition salts.

2. 2-[2'-hydroxy-3'-(1,1-dimethylpropylamino)-propoxy]-β-phenylpropiophenone-hydrochloride.

3. Process for the preparation of 2-[2'-hydroxy-3'-(1,1-dimethylpropylamino)-propoxy]-β-phenylpropiophenone of the formula I

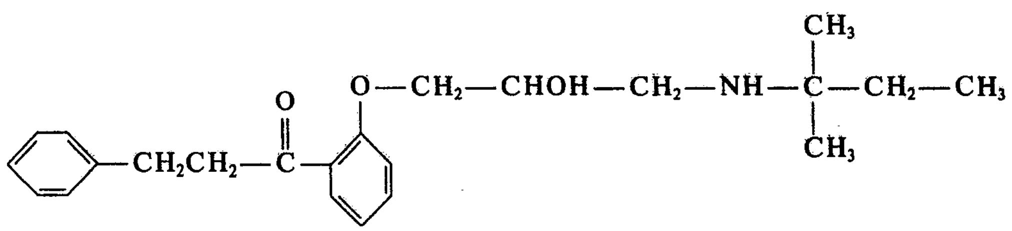

(I)

and its acid addition salts, characterized in that a phenol ether of the general formula II

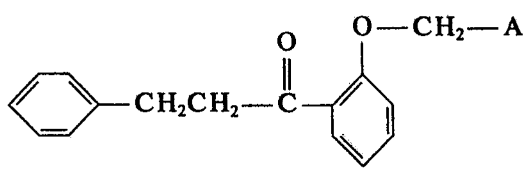

(II)

wherein A means the radical

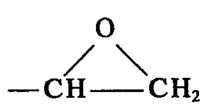

or

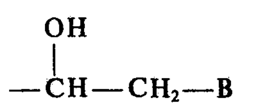

and B is a leaving group, is reacted with the 1,1-dimethylpropylamine of the formula III

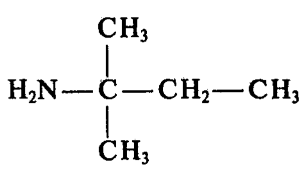

(III)

and optionally the obtained compound of the formula I is resolved into the isomers with an optically active acid and/or is converted into an acid addition salt by reaction with an acid.

4. Pharmaceutical composition for the treatment of arrhythmia, characterized by a content of 2-[2'-hydroxy-3'-(1,1-dimethylpropylamino)-propoxy]-β-phenylpropiophenone or its physiologically compatible acid addition salts.

**Claims for the contracting state AT**

1. Process for the preparation of 2-[2'-hydroxy-3'-(1,1-dimethylpropylamino)-propoxy]-β-phenylpropiophenone of the formula I

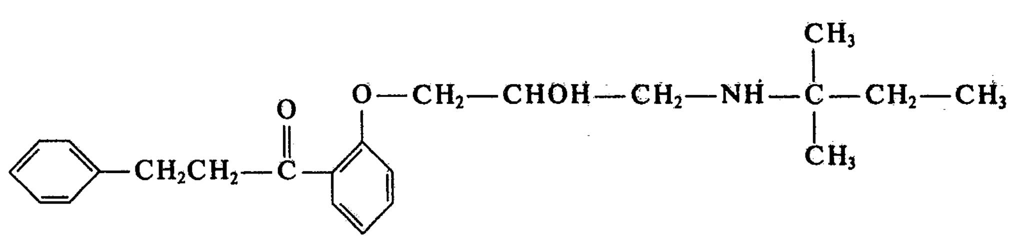

(I)

and its acid addition salts, characterized in that a phenol ether of the general formula II

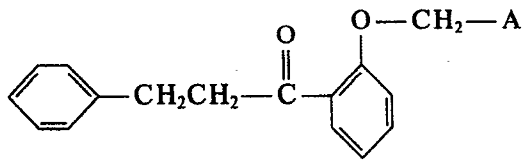

(II)

wherein A means the radical

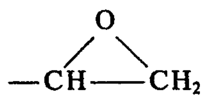 or 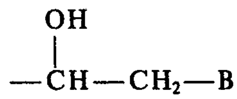

and B is a leaving group, is reacted with the 1,1-dimethylpropylamine of the formula III

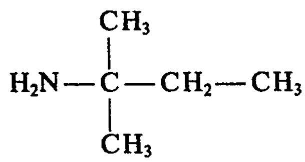

(III)

and optionally the obtained compound of the formula I is resolved into the isomers with an optically active acid and/or is converted into an acid addition salt by reaction with an acid.

2. Pharmaceutical composition for the treatment of arrhythmia, characterized by a content of 2-[2'-hydroxy-3'-(1,1-dimethylpropylamino)-propoxy]-β-phenylpropiophenone or its physiologically compatible acid addition salts.

**Revendications pour les etats contractants: BE, CH/LI, DE, FR, GB, IT, LU, NL, SE**

1. 2-[2'-Hydroxy-3'-(1,1-diméthylpropylamino)-propoxy]-β-phénylpropiophénone répondant à la formule I

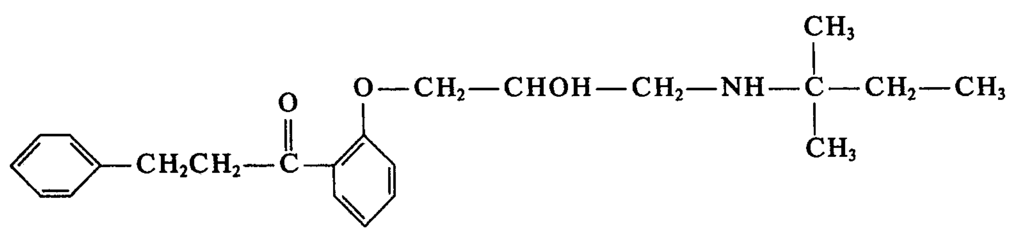

(I)

et ses sels d'addition aux acides.

2. Chlorhydrate de 2-[2'-hydroxy-3'-(1,1-diméthylpropylamino)-propoxy]-β-phénylpropiophénone.

3. Procédé de préparation de la 2-[2'-hydroxy-3'-(1,1-diméthylpropylamino)-propoxy]-β-phénylpropiophénone répondant à la formule I

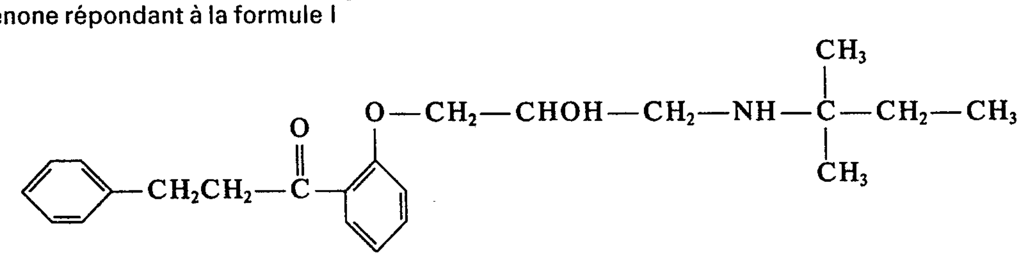

(I)

et de ses sels d'addition aux acides, caractérisé en ce qu'on fait réagir un éther phénolique répondant à la formule générale II

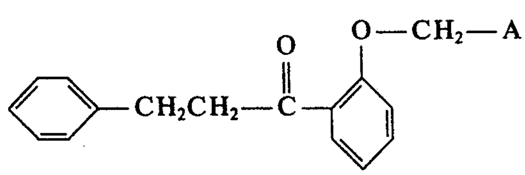

(II)

dans laquelle A désigne les radicaux

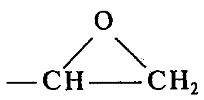 ou 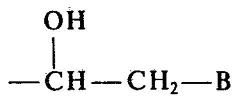

et B est un groupe éliminable nucléofuge, avec la 1,1-diméthylpropylamine répondant à la formule

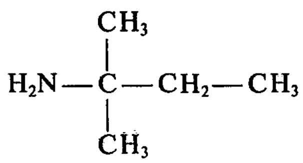 (III)

et en ce qu'on dissocie le cas échéant le composé répondant à la formule I obtenu en ses isomères avec un acide optiquement actif et/ou on le transforme en sel d'addition aux acides par réaction avec un acide.

4. Médicament pour le traitement de troubles du rythme cardiaque, caractérisé en ce qu'il contient de la 2-[2'-hydroxy-3'-(1,1-diméthylpropylamino)-propoxy]-$\beta$-phénylpropiophénone ou un de ses sels d'addition aux acides physiologiquement acceptables.

**Revendications pour l'état contractant AT**

1. Procédé de préparation de la 2-[2'-hydroxy-3'-(1,1-diméthylpropylamino)-propoxy]-$\beta$-phénylpropiophénone répondant à la formule I

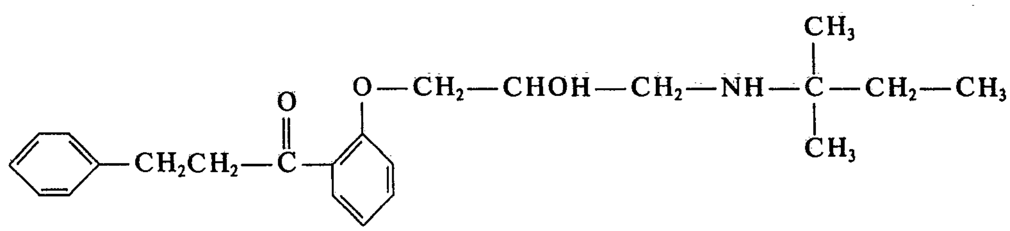 (I)

et de ses sels d'addition aux acides, caractérisé en ce qu'on fait réagir un éther phénolique répondant à la formule générale II

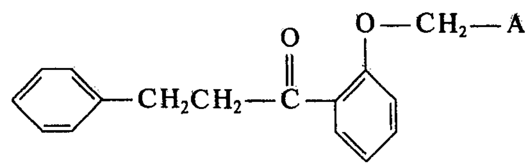 (II)

dans laquelle A désigne le radical

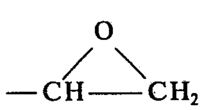 ou 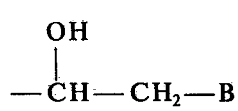

et B est un groupe nucléofuge éliminable, avec la 1,1-diméthylpropylamine répondant à la formule III

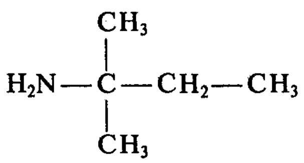 (III)

et le cas échéant on dissocie le composé répondant à la formule I obtenu en ses isomères avec un acide optiquement actif et/ou on le transforme en un sel d'addition aux acides par réaction avec un acide.

2. Médicament pour le traitement des troubles du rythme cardiaque, caractérisé en ce qu'il contient de la 2-[2'-hydroxy-3'-(1,1-diméthylpropylamino)-propoxy]-$\beta$-phénylpropiophénone ou un de ses sels d'addition aux acides physiologiquement acceptables.